# FASCICULE DE BREVET EUROPEEN

(11) **EP 2 874 552 B1**
(45) Date de publication et mention de la délivrance du brevet: **25.05.2016**
(21) Numéro de dépôt: 13756572.7
(22) Date de dépôt: 18.07.2013
(51) Int. Cl.: A61B 17/70

(54) **DISPOSITIF DE GUIDAGE D'UN INSTRUMENT CHIRURGICAL EN POSITION SUR UN ELEMENT D'ANCRAGE OSSEUX COMPRENANT DES MOYENS DE REALIGNEMENT D'UNE TIGE DE LIAISON AVEC L'ELEMENT D'ANCRAGE ET SYSTEME D'INSTRUMENTATION CHIRURGICAL ASSOCIE**
VORRICHTUNG ZUR FÜHRUNG EINES CHIRURGISCHEN INSTRUMENTS IN EINE POSITION AUF EINEM KNOCHENANKERELEMENT MIT EINEM MITTEL ZUR WIEDERAUSRICHTUNG EINES VERBINDUNGSSTABS MIT DEM ANKERELEMENT UND ZUGEHÖRIGES SYSTEM AUS CHIRURGISCHEN INSTRUMENTEN
DEVICE FOR GUIDING A SURGICAL INSTRUMENT INTO POSITION ON A BONE-ANCHOR ELEMENT INCLUDING A MEANS FOR REALIGNING A LINK ROD WITH THE ANCHOR ELEMENT, AND RELATED SYSTEM OF SURGICAL INSTRUMENTS

(30) Priorité: 19.07.2012 FR 1257003
(43) Date de publication de la demande: 27.05.2015
(73) Titulaire: Safe Orthopeadics, 95610 Eragny sur Oise (FR)
(72) Inventeur: PETIT, Dominique, F-62180 Verton (FR)
(74) Mandataire: IP Trust
(86) Numéro de dépôt international: PCT/FR2013/051741
(87) Numéro de publication internationale: WO 2014/013203

(56) Documents cités:
- WO-A2-2010/054079
- US-A1- 2006 079 909
- US-A1- 2010 331 901
- US-A1- 2011 313 464

## Description

### DOMAINE TECHNIQUE DE L'INVENTION

L'invention concerne le domaine de la chirurgie, et plus particulièrement celui de la pose d'implants osseux présentant une tête prévue pour recevoir un bouchon vissable et le cas échéant un accessoire tel qu'une tige de liaison.

L'invention concerne plus particulièrement un dispositif de guidage d'un instrument chirurgical en position sur la tête d'un élément d'ancrage osseux présentant une cavité longitudinale taraudée destinée à recevoir un bouchon vissable, ladite cavité longitudinale débouchant dans un canal transversal apte à recevoir une tige de liaison, l'instrument chirurgical présentant une extrémité d'accouplement sur la tête dudit élément d'ancrage osseux et un canal longitudinal.

### ETAT DE LA TECHNIQUE

La mise en place d'une tige de liaison dans une tête d'un élément d'ancrage osseux reste une opération peu aisée, la tige se trouvant fréquemment déportée (en hauteur et/ou latéralement) vis-à-vis de la tête de l'élément d'ancrage osseux. Il est alors parfois nécessaire d'avoir recours à des instruments complémentaires pour mettre en place la tige de liaison dans ladite tête de l'élément d'ancrage.

Un exemple d'un tel instrument est décrit dans la demande WO2005/099602 lequel divulgue un instrument chirurgical destiné à pousser une tige de liaison dans un implant rachidien.

Il est également connu du brevet US6440133 un instrument chirurgical pour réduire une tige vers un élément d'ancrage osseux. L'instrument comprend un élément venant en prise avec l'élément d'ancrage osseux, ledit élément en prise présentant une partie distale adaptée pour recevoir une partie de la tige de liaison, un élément réducteur disposé de façon mobile autour de l'élément mettant en prise l'élément d'ancrage osseux et un actionneur couplé de façon pivotante à l'élément mettant en prise l'élément d'ancrage osseux et couplé de façon pivotante à l'élément réducteur, l'actionneur permettant de déplacer l'élément réducteur pour que ce dernier entre en contact avec la tige de liaison et déplacer cette dernière vers l'élément d'ancrage osseux. L'élément mettant en prise l'élément d'ancrage osseux comprend une paire de bras flexibles et une patte au niveau de l'extrémité distale de chacun des bras flexibles, chacune desdites pattes définissant un canal pour y recevoir la tige de liaison à l'intérieur.

Un autre exemple d'instrument chirurgical pour réduire une tige est décrit dans la demande US 2010/0331901. L'instrument comprend un tube en prise avec la tête de l'ancrage osseux et les deux extrémités distales du tube sont couplées à l'extérieur de la tête.

WO 2010/054079 décrit également un instrument chirurgical de réduction d'une barre de liaison, instrument comprenant un équipement destiné à être couplé à un élément osseux et un dispositif de réduction composé d'une tige à l'extrémité de laquelle deux pattes sont prévues pour tenir la barre de liaison. Le dispositif de réduction est disposé latéralement par rapport à l'équipement.

Ces instruments présentent cependant un défaut majeur qui est le positionnement sur la tête de l'élément d'ancrage. En effet, un positionnement précis est nécessaire pour réaliser une attache satisfaisante entre l'instrument et l'élément d'ancrage, ce qui requiert un geste minutieux. Les tissus environnant l'incision chirurgicale gênent en effet l'accès à la tête de l'élément d'ancrage et parfois rendent impossible la mise en place de ces instruments de par leur constitution. La mise en place est d'autant plus difficile que les têtes des éléments d'ancrage utilisées sont la plupart du temps mobiles.

L'invention vise à remédier à ces problèmes en proposant un instrument permettant de guider un dispositif de guidage tout en assurant le positionnement de la tige de liaison par rapport à la tête de l'élément d'ancrage osseux, et ce qu'il s'agit d'une tête d'élément fixe ou multiaxiale.

L'invention a également pour objet de proposer un dispositif de guidage facilitant la mise en place de l'instrument chirurgical sur la tête de l'élément d'ancrage osseux.

### OBJET DE L'INVENTION

La présente invention propose un dispositif de guidage d'un instrument chirurgical selon la revendication indépendante 1. Des réalisations avantageuses de l'invention sont décrites dans les revendications dépendantes. L'invention concerne selon son acception la plus générale un dispositif de guidage d'un instrument chirurgical présentant une extrémité d'accouplement sur la tête d'un élément d'ancrage osseux et un canal longitudinal, la tête de l'élément d'ancrage osseux présentant une cavité longitudinale taraudée destinée à recevoir un bouchon vissable, ladite cavité longitudinale débouchant dans un canal transversal apte à recevoir une tige de liaison, caractérisé en ce que ledit dispositif de guidage comprend une tige de guidage de l'instrument chirurgical, d'axe longitudinal A, présentant une extrémité comportant deux pattes semi-tubulaires dont la section extérieure est complémentaire de la section intérieure de ladite cavité longitudinale et dont la section intérieure est au moins égale au diamètre de la tige de liaison, la section transversale du dispositif de guidage étant toujours inférieure à la section du canal longitudinal de l'instrument chirurgical afin de permettre l'introduction et le retrait en direction distale dudit dispositif.

Le dispositif de guidage ainsi agencé permet de guider l'instrument chirurgical sur la tête de l'élément d'ancrage osseux mais également de réaligner longitudinalement la tige de liaison avec la tête de l'élément d'ancrage.

Lorsque la tête de l'élément est mobile, le dispositif de guidage permet d'orienter la tête dans une position où les tissus environnant de l'incision ne gêne pas le geste chirurgical.

Avantageusement, lesdites pattes présentent une section transversale s'inscrivant dans le volume défini entre la paroi intérieure taraudée de ladite cavité longitudinale, et le plan parallèle au plan longitudinal médian et passant par la surface de la tige de liaison lorsque celle-ci est positionnée dans ledit canal transversal.

De préférence, la section intérieure des pattes semi-tubulaires est agencée pour permettre un positionnement longitudinal de la tige lors de l'insertion du dispositif dans la cavité longitudinale. Selon un mode de réalisation particulier, les pattes semi-tubulaires sont prolongées en partie distale par une zone de positionnement longitudinal de la tige de liaison lors de la mise en place du dispositif de guidage dans la tête de l'élément d'ancrage osseux, ladite zone comportant un canal transversal présentant une section intérieure au moins égale au diamètre de la tige de liaison.

Selon une variante particulière, la section intérieure des pattes semi-tubulaires comporte une zone élargie s'étendant en dehors de la cavité longitudinale lors de l'insertion du dispositif dans ladite cavité. Cet élargissement autorise ainsi un débattement latéral de la tige de liaison, ce qui facilite le geste chirurgical en n'imposant pas une position parfaitement alignée de la tige de liaison avec la tête de l'élément d'ancrage.

Avantageusement, la section extérieure des pattes semi-tubulaires comporte un épaulement agencé pour venir en butée sur la tête de l'élément d'ancrage. Cela permet ainsi d'indiquer visuellement et tactilement la mise en place correcte du dispositif de guidage dans la tête de l'élément d'ancrage osseux.

Selon une variante particulière, les pattes semi-tubulaires sont prolongées en direction distale par une zone conique apte à provoquer l'écartement de l'extrémité proximale flexible d'un instrument chirurgical à l'intérieur duquel est introduit ledit dispositif de guidage.

Selon une autre variante, les pattes semi-tubulaires sont flexibles et évasées au repos. Cela permet ainsi d'assurer une fonction de clipsage du dispositif de guidage sur la tête de l'élément d'ancrage osseux, et donc un maintien temporaire de celui-ci sur ladite tête.

L'invention concerne également un système d'instrumentation chirurgical comprenant un instrument chirurgical présentant une extrémité d'accouplement sur la tête d'un élément d'ancrage osseux et un canal longitudinal, la tête de l'élément d'ancrage osseux présentant une cavité longitudinale taraudée destinée à recevoir un bouchon vissable, ladite cavité longitudinale débouchant dans un canal transversal apte à recevoir une tige de liaison caractérisé en ce qu'il comporte en outre un dispositif de guidage comprenant une tige de guidage de l'instrument chirurgical, d'axe longitudinal A, présentant une extrémité comportant deux pattes semi-tubulaires dont la section extérieure est complémentaire de la section intérieure de ladite cavité longitudinale, et dont la section intérieure est au moins égale au diamètre de la tige de liaison, la section transversale du dispositif de guidage étant toujours inférieure à la section du canal longitudinal de l'instrument chirurgical afin de permettre l'introduction et le retrait en direction distale dudit dispositif.

Avantageusement, ledit instrument chirurgical présente à son extrémité deux bras présentant chacune un épaulement s'étendant transversalement sur la surface intérieure du bras, ledit épaulement étant apte à s'accrocher dans une cavité complémentaire prévue sur la tête de l'élément d'ancrage osseux.

Selon une variante particulière, lesdits bras de l'instrument sont flexibles et en ce que ledit dispositif présente une zone tronconique apte à provoquer l'écartement desdits bras flexibles lors de l'insertion du dispositif de guidage à l'intérieur dudit instrument chirurgical. Cela facilite ainsi la mise en place de l'instrument chirurgical sur la tête de l'élément d'ancrage osseux.

Selon une autre variante avantageuse, le dispositif de guidage et l'instrument chirurgical sont réalisés au moins en partie par injection de polymère pour un usage unique.

Le dispositif de guidage est destiné à faire partie d'un kit d'instruments décrit dans la demande WO2011/080426.

### BREVE DESCRIPTION DES FIGURES

D'autres objets et avantages de l'invention apparaîtront au cours de la description qui suit, faite en référence aux dessins annexés, dans lesquels :
- la figure 1 représente une vue du dispositif de guidage selon un premier l'invention et un élément d'ancrage osseux ;
- la figure 2 représente le dispositif de guidage en position sur l'élément d'ancrage osseux ;
- la figure 3 représente une vue en coupe selon l'axe III-III du dispositif de guidage de la figure 2 ;
- la figure 4 représente une vue en coupe longitudinale du dispositif de guidage selon l'invention ;
- les figures 5 à 7 représentent la mise en place et le retrait de l'instrument chirurgical sur l'élément d'ancrage osseux ;
- La figure 8 représente le dispositif de guidage selon un autre mode de réalisation de l'invention, ledit dispositif de guidage étant en prêt à être positionné sur l'élément d'ancrage osseux.

### DESCRIPTION DETAILLEE DES FIGURES

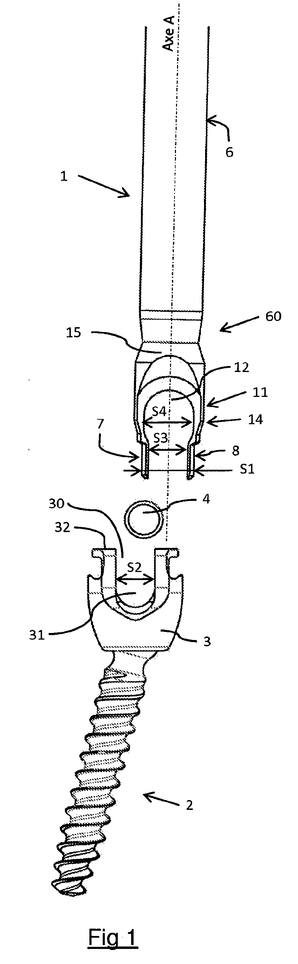

En relation avec les figures 1 à 7, il est décrit un dispositif de guidage 1 d'un instrument chirurgical 5 sur un élément d'ancrage osseux 2 selon un premier mode de réalisation, ledit instrument 5 présentant une extrémité d'accouplement 50 sur la tête 3 de l'élément d'ancrage osseux 2 et un canal longitudinal 51 débouchant.

Dans le mode de réalisation mis en oeuvre, la tête 3 de l'élément d'ancrage osseux 2 présente une cavité longitudinale 30 taraudée destinée à recevoir un bouchon apte à être vissé (non représenté). La tête 3 de l'élément d'ancrage osseux 2 comporte en outre un canal transversal 31 débouchant dans la cavité longitudinale 30, ledit canal transversal 31 étant apte à recevoir une tige de liaison 4.

Dans le mode de réalisation décrit, le dispositif de guidage 1 comprend une tige de guidage 6, d'axe longitudinal A, destiné à guider l'instrument chirurgical 5 lors de sa mise en place sur la tête 3 de l'élément d'ancrage osseux 2, et deux pattes semi-tubulaires 7, 8 ménagées à l'une des extrémités 60 de la tige de guidage 6.

Les deux pattes semi-tubulaires 7, 8 sont conformées pour présenter une section extérieure S1 complémentaire à la section intérieure S2 de la cavité longitudinale 30 d'une part et une section intérieure S3 au moins égale au diamètre D de la tige de liaison 4. Par ailleurs, et comme on le comprendra plus loin, le dispositif de guidage 1 est configuré pour présenter une section transversale toujours inférieure à la section du canal longitudinal 51 de l'instrument chirurgical 5 afin de permettre l'introduction et le retrait en direction distale du dispositif de guidage 1.

Par le terme « distal », on entend la partie ou l'extrémité d'un élément qui est la plus éloignée de l'élément d'ancrage osseux 2 tandis que par le terme « proximal » on entend la partie ou l'extrémité de l'élément qui est le plus proche de l'élément d'ancrage osseux 2.

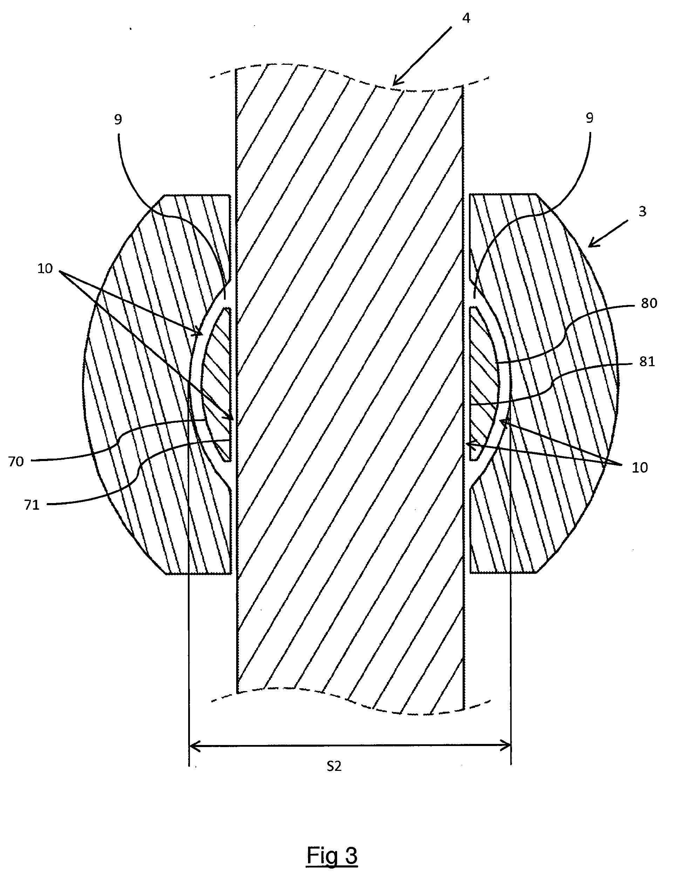

Avantageusement, les pattes semi-tubulaires 7, 8 présentent une section transversale s'inscrivant dans le volume défini entre la surface intérieure taraudée de la cavité longitudinale 30 et le plan parallèle au plan longitudinal médian et passant par la surface de la tige de liaison 4 lorsque celle-ci est positionnée dans le canal transversal 31 (figure 3).

Afin de faciliter le retrait du dispositif de guidage 1 une fois l'instrument chirurgical 5 en place sur la tête 3 de l'élément d'ancrage osseux 2, il est prévu de dimensionner les pattes semi-tubulaires 7, 8 de manière à avoir un léger jeu 10 entre les pattes 7, 8 et la surface intérieure de la cavité longitudinale 30 d'une part et la surface de la tige de liaison 4 d'autre part. A cet égard, et selon une configuration préférée, les pattes semi-tubulaires 7, 8 présentent une distance de 5,5 millimètres mesurée entre leur surface intérieure respective (diamètre standard des tiges) et une distance de 7,5 millimètres mesurée entre leur surface extérieure. Bien entendu, les pattes semi-tubulaires 7, 8 ne sont pas limitées dans ce dimensionnement, celui-ci pouvant être revu selon les dimensions des tiges de liaison et des têtes des éléments d'ancrage osseux mises en oeuvre.

Dans l'exemple illustré sur la figure 3, la surface extérieure 70, 80 des pattes semi-tubulaires 7, 8 est arrondie tandis que la surface intérieure 71, 81 de ces dernières est plane. Selon une variante de réalisation, il peut être également prévu que la surface intérieure des pattes présente une forme concave, les pattes présentant ainsi un profil de section semi-circulaire.

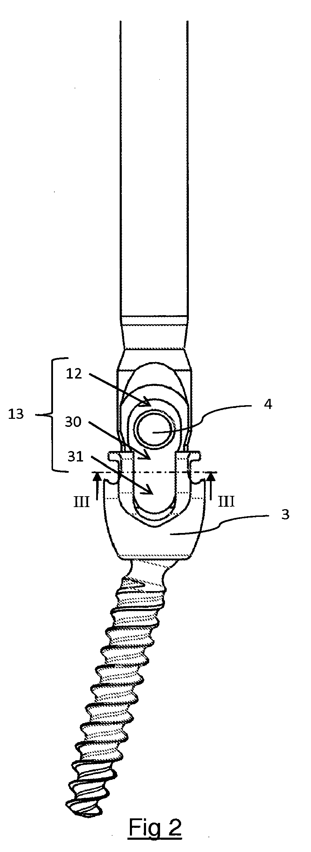

Avantageusement, les pattes semi-tubulaires 7, 8 sont prolongées en partie distale par une zone de positionnement longitudinal 11 de la tige lors du montage du dispositif de guidage 1 sur la tête 3 de l'élément d'ancrage osseux 2. On définit le positionnement longitudinal de la tige de liaison 4 par rapport à l'axe A de cette dernière. La zone de positionnement longitudinal 11 comporte un canal transversal 12 présentant une section intérieure S4 au moins égale au diamètre de la tige de liaison 4. Le canal transversal 12 de la zone de positionnement longitudinal 11 est tel que lorsque le dispositif de guidage 1 est monté sur la tête 3 de l'élément d'ancrage osseux 2, les pattes semi-tubulaires 7, 8 et la zone de positionnement 11 délimitent avec la tête 3 de l'élément d'ancrage osseux 2 une fente oblongue 13 s'étendant suivant l'axe longitudinal AA (figure 2).

Avantageusement, la zone de positionnement 11 présente une section intérieure S4 supérieure au diamètre D de la tige de liaison 4. Cet élargissement de section vis-à-vis de celle des pattes semi-tubulaires 7, 8 a pour avantage de permettre un débattement latéral de la tige de liaison 4 et ainsi de limiter l'effort à appliquer sur ladite tige 4 pour son repositionnement éventuel dans l'alignement du canal transversal 31 lors de la mise en place du dispositif de guidage 1 dans la tête 3 de l'élément d'ancrage osseux 2.

Avantageusement, la section extérieure S1 des pattes semi-tubulaires 7, 8 comporte un épaulement 14 agencé pour venir en butée sur la surface supérieure 32 de la tête 3 de l'élément d'ancrage d'osseux 3. Le terme « supérieur » est défini ici par rapport à la position de l'élément d'ancrage osseux 2 sur les figures. Dans l'exemple illustré, l'épaulement 14 délimite les pattes semi-tubulaires 7, 8 de la zone de positionnement 11 longitudinal de la tige. Ainsi, et comme illustré sur la figure 2, la zone de positionnement 11 s'étend en dehors de la cavité longitudinale 30 de l'élément d'ancrage osseux 2 lorsque le dispositif de guidage 1 est monté dans la tête 3 de l'élément d'ancrage osseux 2.

Selon un autre mode de réalisation, il peut être prévu que l'épaulement vienne en butée sur un chanfrein ménagé en entrée de la cavité longitudinale 30 de la tête 3 de l'élément d'ancrage osseux 2.

Selon une configuration particulière, les pattes semi-tubulaires 7, 8 sont flexibles et évasées au repos. Cela a pour avantage de permettre la retenue du dispositif de guidage 1 une fois celui-ci en position dans la tête 3 de l'élément d'ancrage osseux 2, le jeu 10 précédemment décrit n'existant pas dans cette variante.

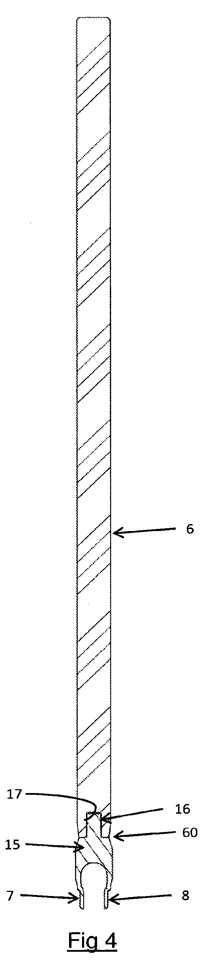

Avantageusement, les pattes semi-tubulaires 7, 8 sont reliées entre elles par une portion de jonction 15 sur laquelle la tige de guidage 6 est fixée. Dans l'exemple illustré sur la figure 4, la fixation de la tige de guidage 6 sur la portion de jonction 15 des pattes semi-tubulaires 7, 8 est réalisée au moyen d'une partie formant tenon 16 ménagée sur la portion de jonction 15 et d'une partie formant mortaise 17 formée au niveau de l'extrémité 60 de la tige de guidage 6. Avantageusement, les parties formant tenon et mortaise 16, 17 sont configurées pour que l'assemblage de la tige de guidage 6 et des pattes semi-tubulaires 7, 8 s'effectue en force. Afin de renforcer la fixation de la tige de guidage 6 sur les pattes semi-tubulaires 7, 8, il peut être prévu d'encoller tout ou partie des parties formant tenon et mortaise 16, 17.

Avantageusement, la partie formant tenon 16 de la portion de jonction 15 comporte des méplats aptes à coopérer avec des méplats ménagés sur la face intérieure de la partie formant mortaise 17 de la tige de guidage 6. Cet arrangement a pour avantage d'empêcher la rotation des pattes semi-tubulaires 7, 8 par rapport à la tige de guidage 6.

L'assemblage est tel qu'une fois assemblés, les deux éléments sont indémontables.

Il est bien entendu évident que l'assemblage des deux éléments pourra être réalisé par tout autre moyen connu de l'homme du métier sans sortir du cadre de l'invention.

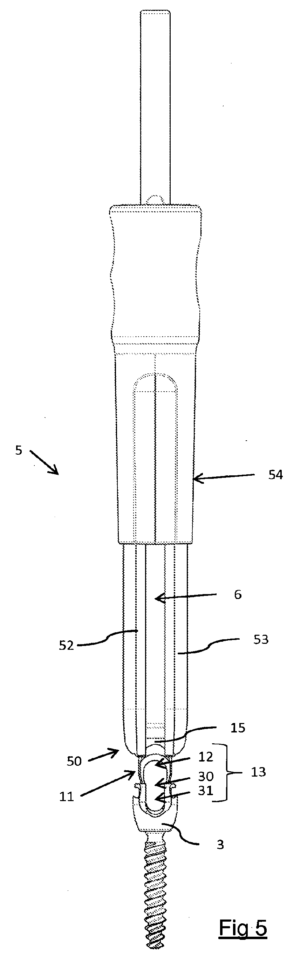

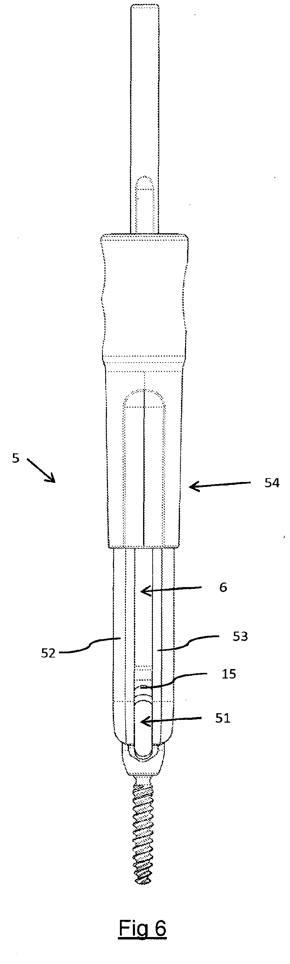

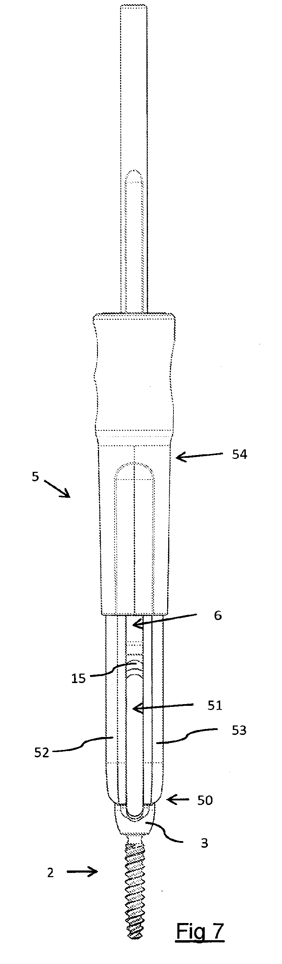

Les figures 5 à 7 représentent les étapes de la mise en place d'un instrument chirurgical 5, se présentant sous la forme d'une tube, sur l'élément d'ancrage osseux 2 (figures 5 et 6) puis du retrait du dispositif de guidage 1 une fois l'instrument chirurgical 5 en position sur la tête 3 de l'élément d'ancrage osseux 2 (figure 7). Dans l'exemple illustré, l'instrument chirurgical 5 comporte deux bras 52, 53 flexibles raccordés l'un à l'autre en partie distale au moyen d'un manchon 54 et agencés l'un par rapport à l'autre pour délimiter un canal longitudinal 51 débouchant de part et d'autre des extrémités de l'instrument 5, ledit canal étant apte à recevoir la tige de guidage 6 du dispositif de guidage 1. Chaque bras 52, 53 présente un épaulement ou tout élément analogue (pion, ergot, etc.) s'étendant transversalement en direction du canal longitudinal 51, ledit épaulement étant apte à s'accrocher dans une cavité ou empreinte de forme complémentaire prévue sur la tête 3 de l'élément d'ancrage osseux 2.

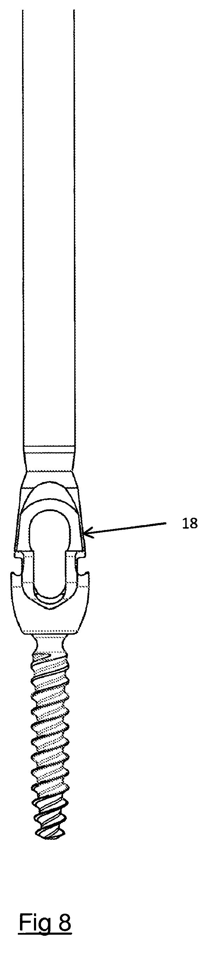

Afin de faciliter la mise en place de l'instrument chirurgical 5, il peut être prévu selon un autre mode de réalisation illustré sur la figure 8, un dispositif de guidage 1 dont les pattes semi-tubulaires 7, 8 sont prolongées en direction distale par une zone tronconique 18. La zone tronconique 18 est disposée de sorte à présenter un évasement suivant la direction proximale. Cette zone conique 18 provoque l'écartement de l'extrémité proximale flexible de l'instrument chirurgical 5 à l'intérieur duquel est introduit ledit dispositif de guidage 1 lors du passage de l'instrument chirurgical 5 sur la zone conique 18.

Avantageusement, le cône présente une base de section supérieure à la section extérieure des bras de l'instrument chirurgical 5. Ainsi, lorsque les bras sont glissés le long de la tige de guidage 6, la zone tronconique 18 va écarter progressivement les bras 52, 53 de l'instrument chirurgical 5. La position légèrement écartées des bras 52, 53 de l'instrument chirurgical 5 ainsi obtenue lorsque ledit instrument atteint la position de clipsage sur la tête 3 de l'élément d'ancrage osseux 2 facilite le clipsage desdits bras 52, 53 sur la tête 3 de l'élément d'ancrage osseux 2.

Avantageusement, le dispositif de guidage 1 et l'instrument chirurgical 5 sont réalisés au moins en partie par injection de polymère pour un usage unique.

Dans le mode de réalisation précédemment, le dispositif de guidage 1 selon l'invention comprend deux éléments distincts : un premier élément formé par la tige de guidage 6 et un deuxième élément formé des pattes semi-tubulaires 7, 8. Dans ce mode de réalisation, la tige de guidage 6 est avantageusement réalisée en polymère tandis que les pattes semi-tubulaires 7, 8 sont réalisées en métal.

Il peut être cependant prévu un dispositif de guidage 1 dont la tige de guidage 6 et les pattes semi-tubulaires 7, 8 sont formées d'un seul tenant sans pour autant sortir du cadre de l'invention.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Dispositif de guidage (1) d'un instrument chirurgical (5) présentant une extrémité d'accouplement sur la tête (3) d'un élément d'ancrage osseux (2) et un canal longitudinal (51), la tête (3) de l'élément d'ancrage osseux (2) présentant une cavité longitudinale (30) taraudée destinée à recevoir un bouchon vissable, ladite cavité longitudinale (30) débouchant dans un canal transversal (31) apte à recevoir une tige de liaison (4), **caractérisé en ce que** ledit dispositif de guidage (1) comprend une tige de guidage (6) de l'instrument chirurgical (5), d'axe longitudinal A, présentant une extrémité (60) comportant deux pattes semi-tubulaires (7, 8) dont la section extérieure (S1) est complémentaire de la section intérieure (S2) de ladite cavité longitudinale (30) et dont la section intérieure (S3) est au moins égale au diamètre (D) de la tige de liaison (4), la section transversale du dispositif de guidage (1) étant toujours inférieure à la section du canal longitudinal (51) de l'instrument chirurgical (5) afin de permettre l'introduction et le retrait en direction distale dudit dispositif.

2. Dispositif de guidage (1) selon la revendication 1, **caractérisé en ce que** les pattes semi-tubulaires (7, 8) présentent une section transversale s'inscrivant dans le volume (9) défini entre la paroi intérieure taraudée de ladite cavité longitudinale (30), et le plan parallèle au plan longitudinal médian et passant par la surface de la tige de liaison (4) lorsque celle-ci est positionnée dans ledit canal transversal 31.

3. Dispositif de guidage (1) selon la revendication 1 ou la revendication 2, **caractérisé en ce que** les pattes semi-tubulaires (7, 8) sont prolongées en partie distale par une zone de positionnement (11) longitudinal de la tige de liaison (4) lors de la mise en place du dispositif de guidage (1) dans la tête (3) de l'élément d'ancrage osseux (2), ladite zone comportant un canal transversal (12) présentant une section intérieure (S4) au moins égale au diamètre (D) de la tige de liaison (4).

4. Dispositif de guidage (1) selon l'une quelconque des revendications 1 à 3, **caractérisé en ce que** la section intérieure (S3) des pattes semi-tubulaires (7, 8) comporte une zone élargie s'étendant en dehors de la cavité longitudinale (30) lors de la mise en place du dispositif dans ladite cavité.

5. Dispositif de guidage (1) selon l'une quelconque des revendications 1 à 4, **caractérisé en ce que** la section extérieure (S1) des pattes semi-tubulaires (7, 8) comporte un épaulement (14) agencé pour venir en butée sur la tête (3) de l'élément d'ancrage.

6. Dispositif de guidage (1) selon l'une quelconque des revendications 1 à 5, **caractérisé en ce que** les pattes semi-tubulaires (7, 8) sont prolongées en direction distale par une zone conique (18) apte à provoquer l'écartement de l'extrémité proximale flexible de l'instrument chirurgical (5) à l'intérieur duquel est introduit ledit dispositif de guidage (1).

7. Dispositif de guidage (1) selon l'une quelconque des revendications 1 à 6, **caractérisé en ce que** les pattes semi-tubulaires (7, 8) sont flexibles et évasées au repos.

8. Système d'instrumentation chirurgical comprenant un instrument chirurgical (5) présentant une extrémité d'accouplement sur la tête (3) d'un élément d'ancrage osseux (2) et un canal longitudinal (51), la tête (3) de l'élément d'ancrage osseux (2) présentant une cavité longitudinale (30) taraudée destinée à recevoir un bouchon vissable, ladite cavité longitudinale (30) débouchant dans un canal transversal (31) apte à recevoir une tige de liaison (4) **caractérisé en ce qu'**il comporte en outre un dispositif de guidage (1) comprenant une tige de guidage (6) de l'instrument chirurgical (5), d'axe longitudinal A, présentant une extrémité comportant deux pattes semi-tubulaires (7, 8) dont la section extérieure (S1) est complémentaire de la section intérieure (S2) de ladite cavité longitudinale (30) et dont la section intérieure (S3) est au moins égale au diamètre (D) de la tige, la section transversale du dispositif de guidage (1) étant toujours inférieure à la section du canal longitudinal de l'instrument chirurgical (5) afin de permettre l'introduction et le retrait en direction distale dudit dispositif.

9. Système d'instrumentation chirurgical selon la revendication précédente, **caractérisé en ce que** ledit instrument chirurgical (5) présente à son extrémité deux bras (52, 53) présentant chacun un épaulement s'étendant transversalement sur la surface intérieure du bras, ledit épaulement étant apte à s'accrocher dans une cavité complémentaire prévue sur la tête (3) de l'élément d'ancrage osseux (2).

10. Système d'instrumentation chirurgical selon la revendication précédente, **caractérisé en ce que** lesdits bras (52, 53) de l'instrument chirurgical (5) sont flexibles et **en ce que** ledit dispositif de guidage (1) présente une zone tronconique (18) apte à provoquer l'écartement desdits bras flexibles lors de l'insertion du dispositif de guidage (1) à l'intérieur dudit instrument chirurgical (5).

11. Système d'instrumentation chirurgical selon l'une quelconque des revendications 8 à 10, **caractérisé en ce que** le dispositif de guidage (1) et l'instrument chirurgical (5) sont réalisés au moins en partie par injection de polymère pour un usage unique.

## Patentansprüche

1. Führungsvorrichtung (1) eines chirurgischen Instruments (5), die ein Endstück zum Ankuppeln an den Kopf (3) eines Knochenverankerungselements (2) sowie einen länglichen Kanal (51) aufweist, wobei der Kopf (3) des Knochenverankerungselements (2) einen längs verlaufenden Hohlraum (30) mit Gewinde aufweist, der dazu bestimmt ist, einen aufschraubbaren Verschluss aufzunehmen, wobei besagter längs verlaufender Hohlraum (30) zu einem quer verlaufenden Kanal (31) führt, welcher in der Lage ist, einen Verbindungsstab (4) aufzunehmen, **dadurch gekennzeichnet, dass** besagte Führungsvorrichtung (1) einen Führungsstab (6) des chirurgischen Instruments (5) mit einer Längsachse A enthält, dessen Ende (60) zwei halbhohle Ansätze (7, 8) aufweist, deren Außenquerschnitt (S1) komplementär zum Innenquerschnitt (S2) besagten längs verlaufenden Hohlraums (30) ausgebildet ist und deren Innenquerschnitt (S3) mindestens gleich dem Durchmesser (D) des Verbindungsstabs (4) ist, wobei der Querschnitt der Führungsvorrichtung (1) immer kleiner ist als der Querschnitt des länglichen Kanals (51) des chirurgischen Instruments (5), um das Einführen und Herausziehen besagter Vorrichtung in distaler Richtung zu ermöglichen.

2. Führungsvorrichtung (1) nach Anspruch 1, **dadurch gekennzeichnet, dass** die halbhohlen Ansätze (7, 8) einen Querschnitt aufweisen, der den Raum (9) zwischen der Innenwand besagten längs verlaufenden Hohlraums (30) mit Gewinde, der parallel zur Längsmittelebene verlaufenden Ebene und der Fläche des Verbindungsstabs (4) umfasst, wenn dieser in besagtem quer verlaufenden Kanal (31) positioniert wird.

3. Führungsvorrichtung (1) nach Anspruch 1 oder Anspruch 2, **dadurch gekennzeichnet, dass** die halbhohlen Ansätze (7, 8) beim Einsetzen der Führungsvorrichtung (1) auf dem Kopf (3) des Knochenverankerungselements (2) distal durch einen länglichen Positionierbereich (11) des Verbindungsstabs (4) verlängert werden, wobei besagter Bereich einen quer verlaufenden Kanal (12) mit einem Innenquerschnitt (S4) aufweist, der mindestens gleich dem Durchmesser (D) des Verbindungsstabs (4) ist.

4. Führungsvorrichtung (1) nach einem der Ansprüche 1 bis 3, **dadurch gekennzeichnet, dass** der Innenquerschnitt (S3) der halbhohlen Ansätze (7, 8) einen erweiterten Bereich aufweist, der sich beim Einsetzen der Vorrichtung in besagten Hohlraum außerhalb des längs verlaufenden Hohlraums (30) befindet.

5. Führungsvorrichtung (1) nach einem der Ansprüche 1 bis 4, **dadurch gekennzeichnet, dass** der Außenquerschnitt (S1) der halbhohlen Ansätze (7, 8) eine Schulter (14) aufweist, die so angeordnet ist, dass sie auf dem Kopf (3) des Verankerungselements zum Anschlag kommt.

6. Verankerungsvorrichtung (1) nach einem der Ansprüche 1 bis 5, **dadurch gekennzeichnet, dass** die halbhohlen Ansätze (7, 8) in distaler Richtung durch einen kegelförmigen Bereich (18) verlängert werden, der in der Lage ist, das bewegliche proximale Ende des chirurgischen Instruments (5), in welches besagte Führungsvorrichtung (1) eingeführt ist, zu spreizen.

7. Führungsvorrichtung (1) nach einem der Ansprüche 1 bis 6, **dadurch gekennzeichnet, dass** die halbhohlen Ansätze (7, 8) beweglich und in der Ruhestellung ausgestellt sind.

8. Chirurgisches Instrumentensystem mit einem chirurgischen Instrument (5), das ein Ende zum Ankuppeln an einen Kopf (3) eines Knochenverankerungselements (2) und einen länglich verlaufenden Kanal (51) umfasst, wobei der Kopf (3) des Knochenverankerungselements (2) einen längs verlaufenden Hohlraum (30) mit Gewinde aufweist, der dazu bestimmt ist, einen aufschraubbaren Verschluss aufzunehmen, wobei besagter länglicher Hohlraum (30) zu einem quer verlaufenden Kanal (31) führt, der in der Lage ist, einen Verbindungsstab (4) aufzunehmen, **dadurch gekennzeichnet, dass** es des weiteren eine Führungsvorrichtung (1) mit einem Führungsstab (6) des chirurgischen Instruments (5) mit einer Längsachse A umfasst, der an einem Ende zwei halbhohle Ansätze (7, 8) aufweist, deren Außenquerschnitt (S1) komplementär zum Innenquerschnitt (S3) besagten längs verlaufenden Hohlraums (30) ausgebildet ist, und deren Innenquerschnitt (S3) mindestens gleich dem Durchmesser (D) des Stabs ist, wobei der Querschnitt der Führungsvorrichtung (1) immer kleiner als der Querschnitt des längs verlaufenden Kanals des chirurgischen Instruments (5) ist, um die Einführung und das Herausziehen der Vorrichtung in distaler Richtung zu ermöglichen.

9. Chirurgisches Instrumentensystem nach dem vorhergehenden Anspruch, **dadurch gekennzeichnet, dass** besagtes chirurgisches Instrument (5) an seinem Ende zwei Arme (52, 53) aufweist, von denen jeder eine Schulter hat, die sich quer über die Innenfläche des Arms erstreckt, und besagte Schulter in einem komplementär ausgebildeten Hohlraum am Kopf (3) des Knochenverankerungselements (2) befestigt werden kann.

10. Chirurgisches Instrumentensystem nach dem vorhergehenden Anspruch, **dadurch gekennzeichnet, dass** besagte Arme (52, 53) des chirurgischen Instruments (5) beweglich sind und dass besagte Führungsvorrichtung (1) einen kegelstumpfförmigen Bereich (18) aufweist, der in der Lage ist, die Spreizung der besagten beweglichen Arme beim Einführen der Führungsvorrichtung (1) in besagtes chirurgisches Instrument (5) herbeizuführen.

11. Chirurgisches Instrumentensystem nach einem der Ansprüche 8 bis 10, **dadurch gekennzeichnet, dass** die Führungsvorrichtung (1) und das chirurgische Instrument (5) mindestens zum Teil durch Polymereinspritzung für einen einmaligen Gebrauch hergestellt werden.

## Claims

1. A device (1) for guiding a surgical instrument (5) having one end for coupling to the head (3) of a bone-anchoring element (2) and a longitudinal channel (51), with the head (3) of the bone-anchoring element (2) having a tapped longitudinal recess (30) intended to receive a screw cap, with said longitudinal recess (30) leading into a transverse channel (31) capable of receiving a link rod (4), **characterised in that** said guiding device (1) comprises a rod (6) for guiding the surgical instrument (5), having a longitudinal axis A, having one end (60) comprising two semi-tubular tabs (7, 8), the outer section (S1) of which matches the inner section (S2) of said longitudinal recess (30), and the inner section (S3) of which is at least equal to the diameter (D) of the link rod (4), with the cross-section of the guiding device (1) being always smaller than the section of the longitudinal channel (51) of the surgical instrument (5), so as to enable the introduction and the withdrawal, along the distal direction, of said device.

2. A guiding device (1) according to claim 1, **characterized in that** the semi-tubular tabs (7, 8) have a cross-section which is inscribed in the volume (9) defined between the tapped inner wall of said longitudinal recess (30), and the plane parallel to the longitudinal median plane and passing through the surface of the link rod (4) when the latter is positioned in said transverse channel (31).

3. A guiding device (1) according to claim 1 or claim 2, **characterized in that** the distal parts of the semi-tubular tabs (7, 8) are extended by a zone of longitudinal positioning (11) of the link rod (4) during the introduction of the guiding device (1) into the head (3) of the bone-anchoring element (2), with said zone having a transverse channel (12) having an inner section (S4) at least equal to the diameter (D) of the link rod (4).

4. A guiding device (1) according to any one of claims 1 to 3, **characterized in that** the inner section (S3) of the semi-tubular tabs (7, 8) has an enlarged area extending outside of the longitudinal recess (30) during the introduction of the device into said recess.

5. A guiding device (1) according to any one of claims 1 to 4, **characterized in that** the outer section (S1) of the semi-tubular tabs (7, 8) comprises a shoulder (14) so arranged as to abut upon the head (3) of the anchoring element.

6. A guiding device (1) according to any one of claims 1 to 5, **characterized in that** the semi-tubular tabs (7, 8) are extended in the distal direction by a conical area (18) adapted to cause the spacing of the flexible proximal end of the surgical instrument (5) inside which said guiding device (1) is introduced.

7. A guiding device (1) according to any one of claims 1 to 6, **characterized in that** the semi-tubular tabs (7, 8) are flexible and flared at rest.

8. A surgical instrumentation system comprising a surgical instrument (5) having one end for coupling to the head (3) of a bone-anchoring element (2) and a longitudinal channel (51), with the head (3) of the bone-anchoring element (2) having a longitudinal recess (30) internally tapped for receiving a screw cap, with said longitudinal recess (30) leading into a transverse channel (31) adapted to receive a link rod (4), **characterized in that** it further comprises a guiding device (1) comprising a rod (6) for guiding the surgical instrument (5), having a longitudinal axis A, having one end comprising two semi-tubular tabs (7, 8), the outer section (S1) of which matches the inner section (S2) of said longitudinal recess (30), and the inner section (S3) of which is at least equal to the diameter (D) of the rod, with the cross-section of the guiding device (1) being always smaller than the section of the longitudinal channel (5), so as to enable the introduction and the withdrawal, along the distal direction, of said device.

9. A surgical instrumentation system according to the preceding claim, **characterized in that** said surgical instrument (5) is provided, at its end, with two arms (52, 53), each having a shoulder extending transversely over the inner surface of the arm, with said shoulder being adapted to get caught in a matching recess provided on the head (3) of the bone-anchoring element (2).

10. A surgical instrumentation system according to the preceding claim, **characterized in that** said arms (52, 53) of the surgical instrument (5) are flexible and **in that** said guiding device (1) has a frustoconical zone (18) able to cause the spacing of said flexible arms upon the insertion of the guiding device (1) into said surgical instrument (5).

11. A surgical instrumentation system according to any one of Claims 8 to 10, **characterized in that** the guiding device (1) and the surgical instrument (5) are at least partially made by injecting a single-use polymer.
